Europäisches Patentamt

European Patent Office

Office européen des brevets

(11) Veröffentlichungsnummer: **0 268 019**
**A1**

## (12) EUROPÄISCHE PATENTANMELDUNG

(21) Anmeldenummer: 87111598.6

(22) Anmeldetag: 11.08.87

(51) Int. Cl.4: **A61B 17/22** , G10K 15/04 , B06B 1/00

(30) Priorität: 13.11.86 DE 3638723

(43) Veröffentlichungstag der Anmeldung:
25.05.88 Patentblatt 88/21

(84) Benannte Vertragsstaaten:
AT BE CH DE ES FR GB GR IT LI LU NL SE

(71) Anmelder: **Messerschmitt-Bölkow-Blohm Gesellschaft mit beschränkter Haftung**
**Robert-Koch-Strasse**
**D-8012 Ottobrunn(DE)**

(72) Erfinder: **Wondrazek, Fritz, Dr.**
**Riegelstrasse 27**
**D-8068 Pfaffenhofen(DE)**
Erfinder: **Diepold, Gisela**
**Schützenstrasse 10**
**D-8034 Germering(DE)**

(54) **Vorrichtung zur Zertrümmerung eines von einem Fluid umgebenen festen Körpers.**

(57) Beschrieben wird eine Vorrichtung zur Zertrümmerung von Nierensteinen mittels akustischer Stoßwellen, die durch das Licht eines Laser induziert werden und die ein Resonator (7) für akustische Wellen auf den Stein (1) fokussiert.

Im Bereich des Resonators (7) ist ein Element (8) vorgesehen, das aus einem das Laserlicht absorbierenden Material besteht, und auf das das Laserlicht gerichtet ist, so daß sich der die akustische Stoßwelle auslösende Plasmazustand an der Oberfläche des Elements (8) ausbildet. Als Material für das Element (8) wird mit Vorteil Platin verwendet.

Dadurch wird erreicht, daß auch unter ungünstigen Umständen mit Sicherheit bei jedem Laserimpuls akustische Stoßwellen erzeugbar sind.

EP 0 268 019 A1

FIG. 1

## Vorrichtung zur Zertrümmerung eines von einem Fluid umgebenen festen Körpers

Die Erfindung bezieht sich auf eine Vorrichtung zur Zertrümmerung eines von einem Fluid umgebenen festen Körpers, insbesondere in einem Lebewesen, gemäß dem Oberbegriff des Patentanspruchs 1.

Eine derartige Vorrichtung ist aus der DE-OS 35 06 249 bekannt, auf die im übrigen hinsichtlich aller hier nicht erläuterter Begriffe ausdrücklich Bezug genommen wird. Bei dieser bekannten Vorrichtung wird ein Laserstrahl mit einer Leistung verwendet, die so groß ist, daß im Fokuspunkt der "Breakdown-Schwellwert" des Fluids überschritten wird. Mit Breakdown wird dabei eine starke Ionisation eines Mediums im Brennpunkt eines Laserstrahls bezeichnet. Die dabei im Fokalbereich umgesetzte Energie steigt durch Kaskadenionisation in etwa exponentiell mit der Zeit an, so daß sich ein heißes Plasma bildet, das stoßartig expandiert und damit eine akustische Stoßwelle induziert.

Mit dieser bekannten Vorrichtung gemäß dem Oberbegriff des Patentanspruchs 1 können beispielsweise Nierensteine ähnlich wie bei der extrakorporalen Stoßwellenlithotrypsie zerstört werden.

Erfindungsgemäß ist jedoch erkannt worden, daß das bei dieser Vorrichtung verfolgte Prinzip, Stoßwellen im körpereigenen Fluid auszubilden, unter bestimmten Umständen Nachteile hat:

Um einen Breakdown in einem Fluid, beispielsweise Wasser, zu erzeugen, ist eine Strahlungsleistung von ca $6,4*10^{13}$ W/m$^2$ erforderlich, so daß der Laserstrahl auf einen vergleichsweise kleinen Bereich fokussiert werden muß.

Beim Zertrümmern von Nierensteinen wird eine Vielzahl von kleinen und kleinsten Teilchen gebildet, die u.a. auch in den Bereich des Laserstrahls eindringen können. Diese Teilchen oder auch bereits vorhandene Schwebteilchen, z.B. sog. Nierengries, können in den Bereich des fokussierten Laserstrahls eindringen und diesen absorbieren, streuen etc., so daß die "Breakdown-Leistung" nicht erreicht wird.

Der Erfindung liegt die Aufgabe zugrunde, eine Vorrichtung gemäß dem Oberbegriff des Patentanspruchs 1 derart weiterzubilden, daß auch unter ungünstigen Bedingungen, wie beispielsweise nach mehreren Nierenstein-Zertrümmerungen nacheinander Stoßwellen mit Sicherheit bei jedem Laserimpuls erzeugbar sind.

Eine erfindungsgemäße Lösung dieser Aufgabe ist mit ihren Weiterbildungen in den Patentansprüchen gekennzeichnet.

Erfindungsgemäß ist der Laserstrahl auf ein Element gerichtet, das aus einem das Laserlicht - linear oder nichtlinear - absorbierenden Material besteht. Damit beginnt bei der erfindungsgemäßen Vorrichtung die Ausbildung des Plasmazustands, dessen Expansion die Stoßwelle auslöst, nicht im Fluid, sondern an der Oberfläche des Elements:

Hierdurch ergeben sich eine Reihe von Vorteilen:

Durch die Verwendung des Elements, an dem die Stoßwelle "kristallisiert", ist eine wesentlich geringere Leistungsdichte erforderlich als bei der bekannten Vorrichtung, bei der die Stoßwelle im Fluid "kristallisiert" wird. Damit ist es im Gegensatz zu der bekannten Vorrichtung nicht mehr erforderlich, den Laserstrahl zu fokussieren; vielmehr genügt es, den beispielsweise aus einem Lichtleiter (Anspruch 14) austretenden Laserstrahl auf das Element "zu richten". Damit kann auf eine verschmutzungsanfällige Fokussierungsoptik verzichtet werden, die beim Stand der Technik darüberhinaus häufig durch die Plasmabildung in unmittelbarer Nähe zerstört werden kann bzw. deren optische Eigenschaften sich ändern können.

Darüberhinaus wird dieser "gerichtete" Laserstrahl mit vergleichsweise großem Durchmesser durch kleine "Schwebteilchen" wie Bruchstücke, Nierengries etc. wesentlich weniger "gestört", d.h. gestreut oder absorbiert als der auf einen kleinen Fokusfleck fokussierte Strahl beim Stand der Technik.

Weiterbildungen der Erfindung sind in den Unteransprüchen angegeben.

Gemäß Anspruch 2 ist vorgesehen, im Bereich des Elements ein Spülfluid, beispielsweise Wasser oder ein medizinisch verträgliches Fluid (Flüssigkeit oder Gas) zuzuführen, das im Bereich des Elements zumindest teilweise aus der Vorrichtung austritt. Die hierdurch hervorgerufene Fluidströmung spült Bruchstücke etc. des zu zertrümmernden Körpers aus dem Bereich der erzeugten Stoßwelle, so daß nachfolgende Stoßwellen nicht gedämpft werden. Auch verhindert die Strömung insbesondere in der im Anspruch 3 gekennzeichneten Weiterbildung zuverlässig die Ablagerung von Bruchstücken, Schwebteilchen etc. an der erfindungsgemäß ausgebildeten Vorrichtung.

Im Anspruch 4 ist beansprucht, daß eine Absaugeinrichtung insbesondere das Spülfluid und die Bruchstücke des zertrümmerten Körpers absaugt. Hierdurch wird erreicht, daß möglichst wenig Spülfluid sowie möglichst wenig Bruchstücke im Lebewesen verbleiben.

Das Element, an dem die Stoßwellen "kristallisieren", kann aus den verschiedensten Materialien bestehen. Besonders vorteilhaft ist es jedoch gemäß Anspruch 5, ein Metall, beispielsweise Platin zu verwenden, da die Lebensdauer eines derartigen "Kristallisations-Elements" sehr hoch ist und durch den Laserstrahl nur wenig Material abgedampft wird. Das im Anspruch 6 gekennzeichnete Material "Edelstahl" verbindet in vorteilhafter Weise Korrosionsbeständigkeit, Lebensdauer und günstige Kosten.

In den Ansprüchen 7 bis 9 sind verschiedene Ausbildungen des Elements, an dem die Stoßwelle entsteht, gekennzeichnet. Die verschiedenen Ausbildungen haben jeweils spezifische Vorteil im Hinblick die Form der entstehenden Stoßwelle, den Schutz des Resonators und des Austrittsendes des Lichtleiters gegen Bruchstücke sowie den Wirkungsgrad der Umsetzung der Energie des Lasers in Stoßwellenenergie, so daß je nach Einsatzfall ein entsprechendes Element gewählt werden kann. Dabei ist es ohne weiteres möglich, die Vorrichtung so auszugestalten, daß die verschiedensten Elementformen eingesetzt werden können (Anspruch 10).

Der Resonator hat in bekannter Weise die Aufgabe, die entstehenden Stoßwellen auf den zu zertrümmernden Körper zu richten. Entsprechend muß die Form des Resonators gewählt werden. Eine bevorzugte Ausgestaltung des Resona tors, bei der die Bündelung mit einem hohen Wirkungsgrad erfolgt, ist in den Ansprüchen 11 und 12 gekennzeichnet.

In den Ansprüchen 13 und 14 sind bevorzugte Ausgestaltungen beschrieben, die die Handhabung sehr erleichtern und den Durchmesser der Vorrichtung deutlich verringern.

Die Erfindung wird nachstehend anhand eines Ausführungsbeispiels unter Bezugnahme auf die Zeichnung näher beschrieben, in der zeigen:

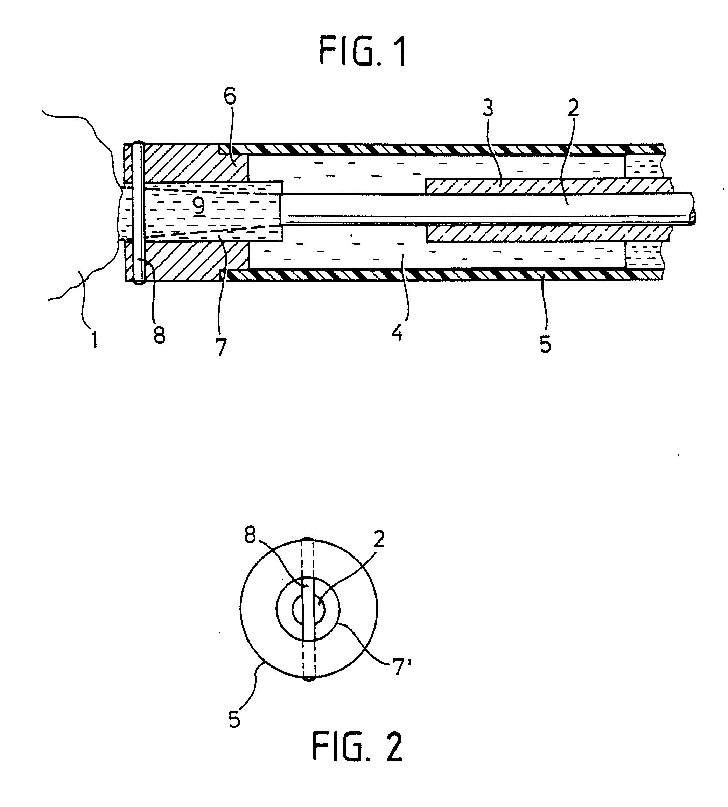

Fig. 1 einen Querschnitt durch eine erfindungsgemäße Vorrichtung, und

Fig. 2 eine Ansicht der in Fig. 1 dargestellten Vorrichtung von vorne.

Die in den Figuren dargestellte Vorrichtung zur Zertrümmerung eines von einem Fluid umgebenen Körpers 1 weist einen Lichtleiter 2 mit einem Lichtleitermantel 3 auf. Der Lichtleiter 2 ist am austrittsseitigen Ende der Vorrichtung von einem Abstandselement 4 gehalten, auf das ein Kunststoffschlauch 5 aufgesteckt ist, der den Lichtleiter 2 konzentrisch umgibt. Mit dem Abstandselement 4 ist ein Halter 6 verbunden, der einen Hohlraum 7 aufweist, in dem ein Resonator 7' für akustische Wellen angeordnet ist bzw. der als Resonator ausgebildet ist, und in den der Lichtleiter 2 mündet. Am vorderen (austrittsseitigen) Ende des Halters 6 ist ein Element 8 befestigt, das bei dem gezeigten Ausführungsbeispiel ein Metalldraht ist und in einem Brennpunkt des Resonators 7' angeordnet ist.

Das Abstandselement 4 ist geschlitzt, so daß ein Spülmittel 9, das durch den Zwischenraum zwischen Lichtleiterman tel 3 und Kunststoffschlauch 5 fließt, in den Hohlraum 7 im Halter 6 austreten kann.

Die beschriebene Vorrichtung arbeitet wie folgt:

Das aus dem Lichtleiter 2 austretende Licht eines Lasers ist auf den Metalldraht 8 gerichtet (nicht fokussiert) und erzeugt an der Oberfläche des Metalldrahts einen Plasmazustand. Das Plasma expandiert und löst in an sich bekannter Weise eine Stoßwelle aus, die von dem Resonator 7' auf den zu zertrümmernden Körper 1 gebündelt wird und diesen zertrümmert. Das Fluid führt Trümmerstücke und eventuell beim Auftreffen des Lasers entstehende Gase ab, so daß der Bereich der Stoßwelle weitgehend frei von Trümmerstücken bleibt.

Vorstehend ist die Erfindung anhand eines Ausführungsbeispiels ohne Beschränkung des allgemeinen Erfindungsgedankens - ein Element vorzusehen, an dem sich die akustischen "Plasma"- Stoßwellen ausbilden - beschrieben worden. Innerhalb dieses allgemeinen Erfindungsgedankens sind selbstverständlich die verschiedensten Modifikationen möglich:

Beispielsweise kann das bei dem dargestellten Ausführungsbeispiel verwendete drahtförmige Element auch durch andere Ausbildungen, beispielsweise gitter-oder folienförmige Elemente ersetzt werden.

Der Resonator 7' kann die Form eines Ellipsenteils haben; ferner kann auch ein sphärischer Resonator verwendet werden.

Darüberhinaus ist es auch möglich, eine Absaugleitung vorzusehen, die insbesondre das zugeführte Fluid und die entstehenden Bruchstücke wieder abführt. Der Austritt des Spül-Fluids kann auch an anderer Stelle erfolgen; beispielsweise kann das Spülfluid aus einer ringförmigen Öffnung austreten, die das Element umgibt.

Ferner ist es auch denkbar, die Vorrichtung in ein Endoskop zu integrieren und/oder mit einem Bildsensor zu versehen, um den Behandlungserfolg überprüfen zu können.

**Ansprüche**

1. Vorrichtung zur Zertrümmerung eines von einem Fluid umgebenen festen Körpers (1), insbesondere in einem Lebewesen, mittels akustischer Stoßwellen, die durch das Licht eines Laser induziert werden und die ein Resonator für akustische Wellen auf den Körper fokussiert, dadurch **gekennzeichnet**, daß im Bereich des Resonators (7') ein Element (8) vorgesehen ist, das aus einem das Laserlicht absorbierenden Material besteht, und auf das Laserlicht gerichtet ist, so daß sich der die akustische Stoßwelle auslösende Plasmazustand an der Oberfläche des Elements ausbildet.

2. Vorrichtung nach Anspruch 1,
dadurch **gekennzeichnet**, daß im Bereich des Elements ein Spülfluid (9) zuführbar ist, das zumindest teilweise aus der Vorrichtung austritt.

3. Vorrichtung nach Anspruch 2,
dadurch **gekennzeichnet**, daß das Spülfluid das Element (8) umfließt.

4. Vorrichtung nach Anspruch 2 oder 3,
dadurch **gekennzeichnet**, daß eine Absaugeinrichtung vorgesehen ist, die insbesondere das Spülfluid und die Bruch stücke des zertrümmerten Körpers absaugt.

5. Vorrichtung nach einem der Ansprüche 1 bis 4,
dadurch **gekennzeichnet**, daß das Element (8) aus Metall besteht.

6. Vorrichtung nach Anspruch 5,
dadurch **gekennzeichnet**, daß das Element (8) aus Edelstahl besteht.

7. Vorrichtung nach einem der Ansprüche 1 bis 6,
dadurch **gekennzeichnet**, daß das Element ein Metalldraht ist.

8. Vorrichtung nach einem der Ansprüche 1 bis 6,
dadurch **gekennzeichnet**, daß das Element ein Metallgitter ist.

9. Vorrichtung nach einem der Ansprüche 1 bis 6,
dadurch **gekennzeichnet**, daß das Element eine Metallfolie ist.

10. Vorrichtung nach einem der Ansprüche 1 bis 9,
dadurch **gekennzeichnet**, daß ein Halter (6) für das Element (8) und den Resonator (7') vorgesehen ist, in den die Spülfluid-Zuleitung sowie ein Lichtleiter (2) enden, und in den das Element (8) auswechselbar eingesetzt ist.

11. Vorrichtung nach einem der Ansprüche 1 bis 10,
dadurch **gekennzeichnet**, daß der Resonator (7') die Form eines Ellipsoid-Teils hat.

12. Vorrichtung nach einem der Ansprüche 1 bis 11,
dadurch **gekennzeichnet**, daß das Element in etwa im Bereich eines Brennpunkts des Resonators für die Stoßwellen angeordnet ist.

13. Vorrichtung nach einem der Ansprüche 1 bis 12,
dadurch **gekennzeichnet**, daß die Vorrichtung in ein Endoskop integriert ist.

14. Vorrichtung nach einem der Ansprüche 1 bis 13,
dadurch **gekennzeichnet**, daß ein Lichtleiter (2) für das Laserlicht vorgesehen ist, den die Spülfluid-Zuleitung sowie weitere gegebenenfalls vorgesehene Leitungen, wie Absaugleitungen etc. insbesondere umgeben.

# FIG. 1

# FIG. 2

10 152

| | **EINSCHLÄGIGE DOKUMENTE** | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A,D | EP-A-0 192 833 (WONDRAZEK et al.)<br>--- | | A 61 B 17/22<br>G 10 K 15/04<br>B 06 B 1/00 |
| A | DE-A-2 538 960 (DREYER et al.)<br>* Figur 1; Seite 5, Absatz 1; Seite 7, Absatz 2; Seite 8, Absatz 1 *<br>----- | | |
| | | | **RECHERCHIERTE SACHGEBIETE (Int. Cl.4)**<br><br>A 61 B<br>G 10 K |

Der vorliegende Recherchenbericht wurde für alle Patentansprüche erstellt

| Recherchenort | Abschlußdatum der Recherche | Prüfer |
|---|---|---|
| DEN HAAG | 05-02-1988 | FISCHER G.H. |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus andern Gründen angeführtes Dokument
.............................................................
& : Mitglied der gleichen Patentfamilie, übereinstimmendes
   Dokument

EPO FORM 1503 03.82 (P0403)